# EUROPÄISCHE PATENTSCHRIFT

(11) **EP 1 286 981 B1**
(45) Veröffentlichungstag und Bekanntmachung des Hinweises auf die Patenterteilung: **04.08.2004**
(21) Anmeldenummer: 01940421.9
(22) Anmeldetag: 09.05.2001
(51) Int. Cl.: C07D 307/77, C07D 307/83

(54) **VERFAHREN ZUR HERSTELLUNG VON 2-COUMARON UND SUBSTITUIERTEN 2-COUMARONEN**
METHOD FOR THE PRODUCTION OF 2- COUMARONE AND SUBSTITUTED 2-COUMARONES
PROCEDE DE PRODUCTION DE 2-COUMARONE ET DE 2-COUMARONES SUBSTITUEES

(30) Priorität: 06.06.2000 AT 983002000
(43) Veröffentlichungstag der Anmeldung: 05.03.2003
(60) Teilanmeldung: 04007390.0
(73) Patentinhaber: DSM Fine Chemicals Austria Nfg GmbH & Co KG, 4021 Linz (AT)
(72) Erfinder: STANEK, Michael, A-4020 Linz (AT); HILDEBRAND, Peter, A-4020 Linz (AT); ZIMMERMANN, Curt, A-4312 Ried in der Riedmark (AT); CASTELIJNS, Marianne, NL-6176 JB Spaubeek (NL)
(74) Vertreter: Lindinger, Ingrid
(86) Internationale Anmeldenummer: PCT/EP2001/005235
(87) Internationale Veröffentlichungsnummer: WO 2001/094329

(56) Entgegenhaltungen:
- US-A- 5 616 733
- US-A- 5 773 632

## Beschreibung

2-Coumaron stellt einen wichtigen Rohstoff zur Synthese von Agrochemikalien dar. Aus der Literatur sind bereits verschiedene Verfahren zur Herstellung von 2-Coumaron, das auch als 3H-2-Benzofuran-2-on oder 2-Coumaranon bezeichnet wird, bekannt. Sie beruhen teils auf mehrstufiger Synthese und teils auf katalytischer Dehydrierung in der Gasphase.

R. W. Layer et al. (US 3 862 133) beschreiben die Synthese zur Herstellung von γ-Lactonen der o-Hydroxyphenylessigsäure, wobei der Aromat Substituenten trägt. Im Falle der Verwendung von Phenol und Glyoxal werden nur sehr geringe Ausbeuten an 2-Coumaron erhalten, da auf Grund der fehlenden Substituenten am Aromaten mehrere reaktive Positionen zu vielen Nebenprodukten führen.

Ebenfalls substituierte 3H-2-Benzofuran-2-one werden laut Beschreibung von Pfitzer Inc. (GB 1 337 507) ausgehend von substituierter 2-Methoxy-phenylessigsäure hergestellt. Die Synthese der 2-Methoxy-phenylessigsäure, anschließende Etherspaltung und Cyclisierung zu 2-Coumaron stellt kein kostengünstiges Verfahren dar.

Eine andere Methode zur Herstellung von 5-Chloro-3-H-benzofuran-2-on beschreiben J. C. Vallejos et al (FR 2 686 880), wobei von p-Chlorphenol und Glyoxylsäure in Anwesenheit von Phosphinsäure und katalytischen Mengen an Jod oder Salzsäure ausgegangen wird.

Andere Methoden zur Herstellung von 2-Coumaron sind zwar in der Literatur erwähnt, haben aber nie den Stellenwert einer industriellen Syntheseroute erreicht: T. Fukagawa et al. berichten über die intramolekulare Acyloxylation der Phenylessigsäure (J. Org. Chem., 1982, 47, 2491); E. Baciocchi et al. verwenden trans-2,3-Dichloro-2,3-dihydrobenzofuran als Edukt (J. Org. Chem., 1979, 44, 32) und H. E. Holmquist beschreibt eine Synthese ausgehend von o-Kresol und Kohlenmonoxid. Unlängst beschrieben J. C. Vallejos et al. (US 5 616 733) ein Verfahren zur Herstellung von 2-Coumaron durch katalytische Dampfphasen-Dehydrierung des Kondensationsproduktes aus Cyclohexanon und wäßriger Glyoxylsäure, das vorerst in konzentrierter Essigsäure gelöst wird. Der größte Nachteile dieses Verfahrens liegt neben der raschen Katalysatordeaktivierung darin, dass nur sehr geringe Ausbeuten an 2-Coumaron erhalten werden.

In einer weiteren Literaturstelle beschreiben J. C. Vallejos et al. (US 5 773 632), dass durch Synthese des Enol-Lactons des Kondensationsproduktes aus Cyclohexanon und wäßriger Glyoxylsäure und anschließender katalytischer Gasphasen-Dehydrierung die Ausbeute an 2-Coumaron gesteigert werden kann. Die Herstellung des Kondensationsproduktes aus Cyclohexanon und 50 %iger wäßriger Glyoxylsäure bedarf nachteilig der Verwendung von zusätzlichem Wasser und Salzsäure, die nach erfolgter Reaktion neben dem Überschuß an Cyclohexanon abdestilliert werden müssen. In einem weiteren Syntheseschritt erfolgt dann die Enol-Lactonisierung des Reaktionsgemisches, bestehend aus 2-Oxo-cyctohexyliden-essigsäure (cis und trans) und dem Enol-Lacton der 2-Oxo-cyclohexyliden-essigsäure Das hauptsächlich gebildete Enol-Lacton der 2-Oxo-cyclohexyliden-essigsäure wird nach Vakuumdestillation der Dampfphasen-Dehydrierung ausgesetzt.

Unerwarteterweise konnte ein verbessertes Verfahren zur Herstellung von 2-Coumaron und substituierten 2-Coumaronen durch katalytische Gasphasen-Dehydrierung der Reaktionsprodukte aus Cyclohexanon oder substituierten Cyclohexanonen und einem carboxylhältigen Acylierungsmittel gefunden werden, bei welchem nicht nur die Reaktionsprodukte aus Cyclohexanon oder substituierten Cyclohexanonen und dem carboxylhältigen Acylierungsmittel, sondern auch 2-Coumaron und die substituierten 2-Coumarone in hohen Ausbeuten erhalten werden.

Gegenstand der Erfindung ist daher ein Verfahren zur Herstellung von 2-Coumaron oder substituierten 2-Coumaronen, das dadurch gekennzeichnet ist, dass Cyclohexanon oder substituierte Cyclohexanone der Formel (I) 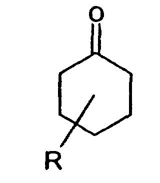 in der R H oder ein C₁-C₃-Alkyl-oder Alkoxyrest ist,
mit dem carboxylhältigen Acylierungsmittel der allgemeinen Formel (II) 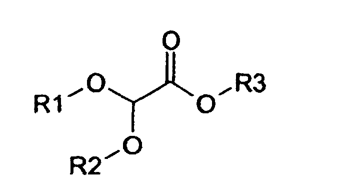 in der R1 und R2 unabhängig voneinander H, oder ein C₁-C₃-Alkylrest sein können und R3 einen C₁-C₃-Alkylrest bedeutet,
a) zu 2-(2-Oxo-cyclohexyl)-2-hydroxy-essigsäurealkylester oder substituierten 2-(2-Oxo-cyclohexyl)-2-hydroxy-essigsäureester umgesetzt werden, die
   a₁) entweder direkt durch katalytische Gasphasen-Dehydrierung in 2-Coumaron oder substituierte 2-Coumarone überführt werden, oder
   a₂) durch azeotrope Destillation unter Verwendung einer Säure oder einem sauren Ionentauscher zu einem Gemisch aus 2-Oxocyclohexyliden-essigsäurealkylester und dem Enol-Lacton der 2-Oxocyclohexyliden-essigsäure oder einem Gemisch aus substituiertem 2-Oxocyclohexyliden-essigsäurealkylester und dem Enol-Lacton der substituierten 2-Oxocyctohexyliden-essigsäure dehydratisiert wird, das anschließend wiederum durch katalytische Gasphasen-Dehydrierung in 2-Coumaron oder substituierte 2-Coumarone überführt wird, oder
b) direkt unter sauren Bedingungen zu einem Gemisch aus 2-Oxocyclohexyliden-essigsäurealkylester und dem Enol-Lacton der 2-Oxo-cyctohexyliden-essigsäure oder einem Gemisch aus substituiertem 2-Oxocyclohexyliden-essigsäurealkylester und dem Enol-Lacton der substituierten 2-Oxocyclohexyliden-essigsäure umgesetzt wird, das anschließend wiederum durch katalytische Gasphasen-Dehydrierung in 2-Coumaron oder substituierte 2-Coumarone überführt wird.

Bei dem erfindungsgemäßen Verfahren wird von Cyclohexanon oder substituierten Cyclohexanonen der Formel (I)

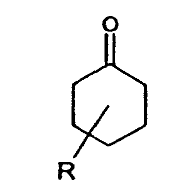

in der R H oder ein C₁-C₃-Alkyl-oder Alkoxyrest ist,
und einem carboxylhältigen Acylierungsmittel der allgemeinen Formel (II) 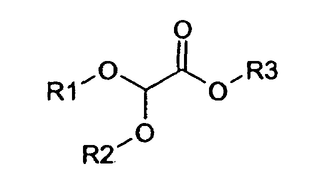 ausgegangen, wobei R1 und R2 unabhängig voneinander H oder ein Methyl-, Ethyloder Propyl-Rest und R3 ein Methyl-, Ethyl- oder Propyl-Rest sein können.
Beispiele für Verbindungen der Formeln II sind Glyoxylsäuremethylestermethylhemiacetal, Glyoxylsäuremethylester-dimethylacetal, Glyoxylsäureethylesterethylhemiacetal u.s.w..
Als carboxylhältiges Acylierungsmittel können auch Gemische von Verbindungen der Formel II verwendet werden.

Der Reaktionsverlauf ist aus dem nachfolgenden Formelschema ersichtlich - R steht für Methyl, Ethyl, Propyl, Methoxy, Ethoxy oder H:

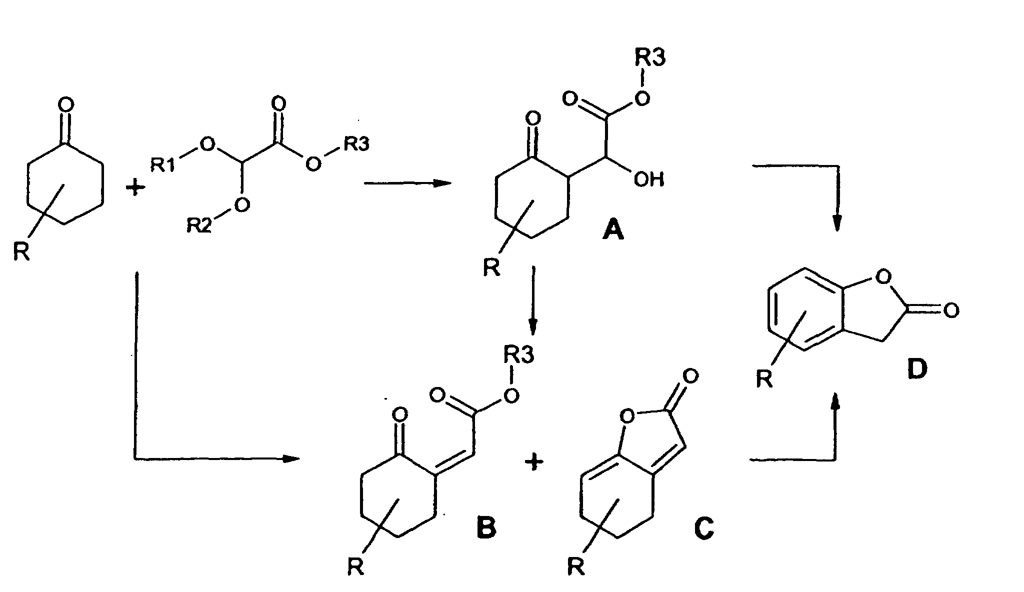

Das Reaktionsprodukt A, der 2-(2-Oxo-cyclohexyl)-2-hydroxy-essigsäurealkyl-ester oder substituierte 2-(2-Oxo-cyclohexyl)-2-hydroxy-essigsäurealkyl-ester, wird erfindungsgemäß nahezu quantitativ durch Umsetzung von Cyclohexanon oder substituierten Cyclohexanonen und dem carboxylhältigen Acylierungsmittel unter inerter Atmosphäre erhalten, wobei vorzugsweise das carboxylhältige Acylierungsrnittel zu Cyclohexanon oder dem substituierten Cyclohexanon langsam zudosiert und der dabei anfallende Alkohol (Methanol, Ethanol, Propanol) über Kopf aus dem Reaktionsgemisch beseitigt wird. Das molare Verhältnis zwischen Cyclohexanon oder dem substituierten Cyclohexanon und dem carboxylhältigen Acylierungsmittel wird zwischen 100:1 und 0,5 :1 gewählt. Bevorzugt beträgt das molare Verhältnis zwischen Cyclohexanon oder dem substituierten Cyclohexanon und dem carboxylhältigen Acylierungsmittel zwischen 20:1 und 1:1, besonders bevorzugt zwischen 10: 1 und 1,5:1. Die Temperatur der Reaktionsmischung beträgt 50 bis 190 °C, bevorzugt 80 bis 150 °C und besonders bevorzugt 95 bis 145 °C. Das Ende der Reaktion wird durch eine geeignete Inprozeß-kontrolle auf das Abreagieren des carboxylhältigen Acylierungsmittels festgestellt. Nach erfolgter Reaktion wird überschüssiges Cyclohexanon oder substituiertes Cyclohexanon unter vermindertem Druck aus dem Reaktionsgemisch entfernt. Das Reaktionsprodukt A kann gegebenenfalls unter Vakuum destilliert werden, wobei bevorzugt bei einem Vakuum < 0.5 mbar destilliert wird.
Das Reaktionsprodukt 2-(2-Oxo-cyclohexyl)-2-hydroxy-essigsäurealkytester (= Reaktionsprodukt A) ist zwar aus der Literatur bekannt und wurde bisher entweder durch enzymatische Reduktion des entsprechenden 2-Oxo-Säureesters (A. Schummer et al, Tetrahedron, Vol. 47, 1991, Seite 9019; S. Tsuboi et al, J. Org. Chem., 1987, 52, 1359; S. Tsuboi et al, Tetrahedron Letters, Vol. 27, 1986, Seite 1915), oder durch Reaktion von Cyclohexanon, sekundärem Amin und Glyoxylsäure in einer Art Mannich Reaktion und anschließender Veresterung erhalten (J. Schreiber et al, Bull. Soc. Chim. Fr., 1973, 2, Seite 625). Die Herstellung in einem einzigen Syntheseschritt aus billigen Rohstoffen war bisher jedoch nicht bekannt und ist deshalb ein weiterer Gegenstand der vorliegenden Erfindung.

Bei dem erfindungsgemäßen Verfahren wird in Schritt a₂) das Reaktionsprodukt A nach Zugabe eines organischen Lösungsmittels und gegebenenfalls einer Säure oder sauren Ionentauschers durch Wasserauskreisen dehydratisiert, wobei ein Gemisch aus 2-Oxocyclohexyliden-essigsäurealkylester (Reaktionsprodukt B, cis- oder trans-Form) und dem Enol-Lacton der 2-Oxo-cyclohexyliden-essigsäure (Reaktionsprodukt C) oder einem Gemisch aus substituiertem 2-Oxocyclohexyliden-essigsäurealkylester (Reaktionsprodukt B, cis- oder trans-Form) und dem substituierten Enol-Lacton der 2-Oxo-cyctohexyliden-essigsäure (Reaktionsprodukt C) erhalten wird. Je nach Reaktionsführung, aber auch durch Zudosierung von Wasser, kann das Verhältnis zwischen B und C gesteuert werden. Die Reaktionstemperatur beträgt mehr als 50 °C, besonders bevorzugt zwischen 80 und 160 °C. Als Säuren eignen sich insbesondere diejenigen, die in einem organischen Medium löslich sind, wobei bevorzugt p-Toluolsulfonsäure, Methansulfonsäure, konzentrierte Schwefelsäure, etc., eingesetzt werden, jedoch besonders bevorzugt wird ein stark saurer lonentauscher verwendet. Als organische Lösungsmittel eignen sich bevorzugt aliphatische oder aromatische, gegebenenfalls halogenierte Kohlenwasserstoffe wie etwa Benzol, Toluol, Xylole und Tetrachlorkohlenstoff, aber auch andere organische Lösungsmittel, die laut klassischer Literatur zum Auskreisen von Wasser dienen.
Die nichtsubstituierten Reaktionsprodukte B und C sind in der Literatur durch Synthese über eine andere, aber wirtschaftlich nicht effiziente Herstellungsroute, als die erfindungsgemäße Variante, bekannt: A. Mondon et al, Chem. Ber., 96, 826, 1963; K. W. Rosenmund et al, Arch. Pharmarz. Ber. dtsch. pharmaz. Ges, 287, 441; A. W. Noites et al, Rec. Trav. Chim., 80, 1334, 1961; Y. Arbuzov et al, Zhur. Obshch. Khim., 32, 3676, 1962, M. N. Kolosov et al, Zhur. Obshch. Khim., 32, 2983, 1962; F. Bonadies et al, Gazz. Chim. Ital., 113, 421, 1983.

Das Gemisch der Reaktionsprodukte B und C kann erfindungsgemäß aber auch in einem einzigen Syntheseschritt (Schritt b) durch Umsetzung von Cyclohexanon oder subsituiertem Cyclohexanon und dem carboxylhältigen Acylierungsmittel unter sauren Bedingungen und unter Schutzgasatmosphäre erhalten werden, wobei vorzugsweise das carboxylhältige Acylierungsmittel zu Cyclohexanon oder dem substituierten Cyclohexanon, gegebenenfalls in Kombination mit einem organischen Lösungsmittel, langsam zudosiert wird. Im Reaktionsgemisch anfallender Alkohol (Methanol, Ethanol, Propanol) kann über Kopf abdestilliert werden. Das molare Verhältnis zwischen Cyclohexanon oder substituiertem Cyclohexanon und dem carboxylhältigen Acylierungsmittel wird zwischen 100:1 und 0,5:1 gewählt. Bevorzugt beträgt das molare Verhältnis 20:1 und 1:1, besonders bevorzugt zwischen 10:1 und 1;5 zu 1. Als organische Lösungsmittel eignen sich besonders bevorzugt aliphatische oder aromatische, gegebenenfalls halogenierte Kohlenwasserstoffe wie etwa Benzol, Toluol, Xylole, Tetrachlorkohlenstoff u.s.w., aber auch Alkohole, wie Methanol, Ethanol, u.s.w.. Wird ein organisches Lösungsmittel zugesetzt, so beträgt das Volumsverhältnis zwischen organischem Lösungsmittel und Cyclohexanon oder dem substituierten Cyclohexanon zwischen 100:1und 0,2 :1, bevorzugt zwischen 50:1 und 0,5:1 und besonders bevorzugt zwischen 10:1 und 1:1.
Als Säuren eignen sich sowohl anorganische als auch organische Säuren, die in äquimolarer Menge oder im molaren Über- oder Unterschuss bezogen auf das carboxylhältige Acylierungsmittel eingesetzt werden können. Insbesondere bevorzugt werden Salzsäure, Schwefelsäure, Phosphorsäure, p-Toluolsulfonsäure, Methansulfonsäure, u.s.w. Besonders bevorzugt wird ein stark saurer Ionentauscher verwendet. Die Temperatur der Reaktionsmischung beträgt 50 bis 180 °C, bevorzugt 80 bis 150 °C und besonders bevorzugt 120 bis 135 °C. Das Ende der Reaktion wird durch eine geeignete Inprozeßkontrolle auf das Abreagieren des carboxylhältigen Acylierungsmittels festgestellt.
Nach erfolgter Reaktion wird überschüssiges Cyclohexanon oder überschüssiges substituiertes Cyclohexanon und bei Bedarf auch das organische Lösungsmittel unter vermindertem Druck aus dem Reaktionsgemisch entfernt. Das Gemisch der Reaktionsprodukte B und C kann gegebenenfalls unter Vakuum destilliert werden, wobei bevorzugt bei einem Vakuum < 500 mbar destilliert wird.

Das erfindungsgemäße Verfahren ist weiters dadurch gekennzeichnet, dass entweder das Reaktionsprodukt A oder das Gemisch der Reaktionsprodukte B und C entweder als Rohprodukt oder isoliert, gegebenenfalls in Kombination mit Methanol, Ethanol oder Diethylether, mit Hilfe eines Trägergases verdampft und katalytisch dehydriert wird, wobei als Dehydrierungskatatysatoren, jene, die dem Stand der Technik zur Aromatisierung von cyclischen Kohlenwasserstoffen entsprechen, verwendet werden, die beispielsweise aus J. Wiley Interscience, New-York, 1985; Houben-Weyl, Phenole, Vol. 2, Georg Thieme- Stuttgart, 1976, 701-716 und J. H. Sinfelt in J. R. Anderson, M. Boudart: Catalysis Science and Technology, Vol. 1, Springer-Verlag, New York 1981 u.s.w. bekannt sind.
Wird Methanol, Ethanol oder Diethylether zugesetzt, so kann das molare Verhältnis zwischen dem Reaktionsprodukt A oder dem Gemisch der Reaktionsprodukte B und C und Methanol, Ethanol oder Diethylether zwischen 1:0,2 bis 100:0,2 gewählt werden, wobei ein molares Verhältnis zwischen 1:1 und 20:1 bevorzugt wird. Besonders bevorzugt ist ein molares Verhältnis zwischen 2:1 und 10:1.

Die katalytische Gasphasendehydrierung erfolgt bei einer Temperatur größer als 140 °C. bevorzugt bei einer Temperatur zwischen 200 und 300 °C und besonders bevorzugt bei einer Temperatur zwischen 220 und 270 °C. Als Katalysator wird bevorzugt Palladium oder Platin auf einem festen Träger, wie zum Beispiel Aluminium-oxid, der bevorzugt eine spezifische Oberfläche von mehr als 1 m²/g hat, in einer Konzentration zwischen 0.5 und 6 % verwendet.
Nach erfolgter Dehydrierung wird das in Kühlfallen gesammelte Kondensat destilliert, um 2-Coumaron oder substituierte 2-Coumarone in hohen Ausbeuten in reiner Form zu isolieren.

Das erfindungsgemäße Verfahren eignet sich somit zur Herstellung von Reaktionsprodukten aus Cyclohexanon oder substituierten Cyclohexanonen und einem carboxylhältigen Acylierungsmittel in hohen Ausbeuten von über 90 %, die anschließend in der Dampfphase katalytisch zu 2-Coumaron oder substituierten 2-Coumaronen in hohen Ausbeuten von über 70 % dehydriert werden.
Im Vergleich zum Stand der Technik werden mit dem erfindungsgemäßen Verfahren sehr einfach aus billigen Rohstoffen in nur einem einzigen Syntheseschritt flüssige, stabile Zwischenprodukte erhalten (Reaktionsprodukte A, B und C), die direkt durch katalytische Gasphasen-Dehydrierung zu 2-Coumaron oder substituierten 2-Coumaronen umgesetzt werden können.

### Beispiel 1:

196 g (2 mol) Cyclohexanon wurden in einem Doppelmantelreaktor vorgelegt und auf 125 °C erwärmt. Über einen Zeitraum von 60 Minuten wurden 120 g (1 mol) Glyoxylsäuremethylester-methyl-hemiacetal eingeleitet und entstehendes Methanol durch einen Stickstoffstrom aus dem Reaktionsgemisch ausgetragen. Nach weiteren 30 Minuten Reaktionszeit wurde bei 105 °C überschüssiges Cyclohexanon und geringe Mengen an Methanol unter reduziertem Druck aus dem Reaktionsgemisch entfernt. Durch nahezu quantitative Umsetzung erhielt man das Reaktionsprodukt A in 98 %i-ger Ausbeute mit einem Gehalt von 98 % (GC + HPLC).

### Beispiel 2:

62 g (0,33 mol) Reaktionsprodukt A wurden mit 300 ml Toluol und 6 g Ionentauscher Amberlyst 15 (Aldrich) versetzt. Nach 60 Minuten wurde die azeotrope Destillation beendet und der Ionentauscher abfiltriert. Überschüssiges Toluol wurde unter vermindertem Druck vom Reaktionsgemisch beseitigt, und bei Bedarf wurde das Reaktionsgemisch destilliert (Siedepunkt: 90 °C bei 1 mbar).
Durch quantitative Umsetzung erhielt man ein Gemisch der Reaktionsprodukte B (cis-Form) und C, wobei sich das Gemisch laut HPLC Chromatogramm aus 95 % Reaktionsprodukt B und 4 % Reaktionsprodukt C zusammensetzte.

### Beispiel 3:

196 g (2 mol) Cyclohexanon und 30 ml konzentrierte Salzsäure wurden in einem Doppelmantelreaktor vorgelegt und auf 100 °C erwärmt. Über einen Zeitraum von 60 Minuten wurden 120 g (1 mol) Glyoxylsäuremethylester-methyl-hemiacetal eingeleitet, und anschließend die Reaktionsmischung noch 30 Minuten bei 100 °C gekocht. Überschüssiges Cyclohexanon, Methanol, Wasser und Salzsäure wurden unter reduziertem Druck abdestilliert. Das Gemisch der Reaktionsprodukte setzte sich laut HPLC aus 68 % Reaktionsprodukt C, 28 % Reaktionsprodukt B (cis-Form) und 3 % cis-2-Oxocyclohexyliden-essigsäure zusammen. Das Reaktionsgemisch wurde durch Destillation bei einem Siedepunkt von 90 °C und einem Druck von 1 mbar destilliert.
Ausbeute: 94 % des Gemisches der Reaktionsprodukte B und C bezogen auf Glyoxylsäuremethylester-methyl-hemiacetal.

### Beispiel 4:

56.1 g (0.5 mol) 4-Methyl-cyclohexanon und 18 g eines Gemisches aus Glyoxylsäuremethylesters und Glyoxylsäuremethylester-methyl-hemiacetal (ca. 80/20 nach Destillation) wurden in einem Doppelmantelreaktor vorgelegt und kontinuierlich auf 135 °C erwärmt. Der entstehende Methanol wurde unter Normaldruck aus dem Reaktionsgemisch ausgetragen. Nach weiteren 30 Minuten Reaktionszeit wurde bei 100 °C überschüssiges 4-Methyl-cyclohexanon und geringe Mengen an Methanol unter reduziertem Druck aus dem Reaktionsgemisch entfernt.
Durch nahezu quantitative Umsetzung erhielt man 2-(2-Oxo-5-methyl-cyclohexyl)-2-hydroxy-essigsäuremethyl-ester in hoher Ausbeute mit einem Gehalt von 95 % (GC).

### Beispiel 5:

Die Produkte aus den Beispielen 1 bis 3 wurden jeweils mit Methanol im molaren Verhältnis 4 zu 1 verdünnt (das Gemisch der Reaktionsprodukte aus Beispiel 2 auch ohne Methanol) und in einem Verdampfer, der je nach Produkt auf einer Temperatur zwischen 210 bis 270 °C gehalten wurde, bei einer Flußrate von 15 ml/h Stickstoffstrom verdampft. Dieser Dampfstrom wurde in den Reaktorstickstoffstrom mit einer Flußrate von 200 bis 300 ml/h geleitet. Der mit 1 % Pd/Al₂O₃ Katalysator gefüllte, vertikal angeordnete Rohreaktor wurde bei einer Temperatur von 260 °C betrieben. Am Ende des Reaktors wurde der Gasstrom durch mehrere Kühlfallen geleitet und das Rohprodukt in einer mit Acetonitril gefüllten Falle kondensiert.
Die Acetonitrillösung wurde anschließend mittels GC und HPLC analysiert. Bezogen auf die eingesetzte Menge an Edukt aus den Beispielen 1 bis 3 wurden Ausbeuten größer 70 % erzielt. Durch Erhöhung des Gasstromes konnten sogar Ausbeuten von ca. 80 % erzielt werden. Bei Bedarf wurde 2-Coumaron oder die substituierten 2-Coumarone unter reduziertem Druck destilliert, wobei der Gehalt an 2-Coumaron oder substituierten 2-Coumaronen 99.8% betrug (GC + HPLC).

## Patentansprüche

1. Verfahren zur Herstellung von 2-Coumaron oder substituierten 2-Coumaronen, **dadurch gekennzeichnet, dass** Cyclohexanon oder substituierte Cyclohexanone der Formel I 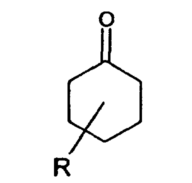 in der R H oder ein C₁-C₃-Alkyl-oder Alkoxyrest ist,
mit einem carboxylhältigen Acylierungsmittel der allgemeinen Formel II 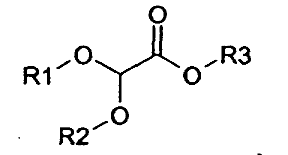 wobei R1 und R2 unabhängig voneinander Wasserstoff oder einen C₁-C₃-Alkyl-Rest und R3 einen C₁-C₃-Alkyl-Rest bedeuten,
a) zu 2-(2-Oxo-cyclohexyl)-2-hydroxy-essigsäurealkylester oder substituierten 2-(2-Oxo-cyclohexyl)-2-hydroxy-essigsäurealkylestern umgesetzt werden, die
a₁) entweder direkt durch katalytische Gasphasen-Dehydrierung in 2-Coumaron oder substituierte 2-Coumarone überführt werden, oder
a₂) durch azeotrope Destillation unter Verwendung einer Säure oder einem sauren Ionentauscher zu einem Gemisch aus 2-Oxocyclohexyliden-essigsäurealkylester und dem Enol-Lacton der 2-Oxo-cyclohexyliden-essigsäure oder einem Gemisch aus substituiertem 2-Oxocyclohexyliden-essigsäurealkylester und dem Enol-Lacton der substituierten 2-Oxocyclohexyliden-essigsäure dehydratisiert wird, das anschließend wiederum durch katalytische Gasphasen-Dehydrierung in 2-Coumaron oder substituierte 2-Coumarone überführt wird, oder
b) direkt unter sauren Bedingungen zu einem Gemisch aus 2-Oxocyclohexyliden-essigsäurealkylester und dem Enol-Lacton der 2-Oxocyclohexylidenessigsäure oder einem Gemisch aus substituiertem 2-Oxocyclohexyliden-essigsäurealkylester und dem Enol-Lacton der substituierten 2-Oxo-cyclohexyliden-essigsäure umgesetzt wird, das anschließend wiederum durch katalytische Gasphasen-Dehydrierung in 2-Coumaron oder substituierte 2-Coumarone überführt wird.

2. Verfahren nach Anspruch 1, **dadurch gekennzeichnet, dass** in Schritt a) oder b) das molare Verhältnis zwischen Cyclohexanon oder substituierten Cyclohexanonen und dem carboxylhältigen Acylierungsmittel zwischen 100 :1 und 0,5 :1 liegt.

3. Verfahren nach Anspruch 1, **dadurch gekennzeichnet, dass** die Reaktionstemperatur in Schritt a) oder b) über 50 °C liegt.

4. Verfahren nach Anspruch 1, **dadurch gekennzeichnet, dass** sich in Schritt a₂) oder b) als Säuren anorganische oder organische Säuren oder ein saurer lonentauscher eingesetzt werden.

5. Verfahren nach Anspruch 4, **dadurch gekennzeichnet, dass** als Säuren p-Toluolsulfonsäure, Methansulfonsäure, Salzsäure, Schwefelsäure oder ein saurer Ionentauscher verwendet werden.

6. Verfahren nach Anspruch 1, **dadurch gekennzeichnet, dass** in Schritt a₂) zur azeotropen Destillation und gegebenenfalls in Schritt b) zum Verdünnen der Reaktionsmischung während der Reaktion, aliphatische oder aromatische, gegebenenfalls halogenierte organische Lösungsmittel eingesetzt werden.

7. Verfahren nach Anspruch 1, **dadurch gekennzeichnet, dass** für die Gasphasendehydrierung in Schritt a₁), a₂) und b) sowohl die entsprechenden rohen Zwischenprodukte, als auch gereinigte Zwischenprodukte eingesetzt werden können.

8. Verfahren nach Anspruch 1, **dadurch gekennzeichnet, dass** die Dampfphasen-Dehydrierung bei einer Temperatur über 140 °C ausgeführt wird.

9. Verfahren nach Anspruch 1, **dadurch gekennzeichnet, dass** bei der Gasphasen-Dehydrierung ein Katalysator bestehend aus Palladium oder Platin, der auf einem inerten, festen Träger mit einer spezifischen Oberfläche von mehr als 1 m²/g, aufgebracht ist, eingesetzt wird.

10. Verfahren zur Herstellung von 2-(2-Oxo-cyclohexyl)2-hydroxyessigsäurealkylester oder substituierten 2-(2-Oxo-cyclohexyl)-2-hydroxyessigsäurealkyl-estern, **dadurch gekennzeichnet, dass** Cyclohexanon oder substituierte Cyclohexanone mit dem carboxylhältigem Acylierungsmittel umgesetzt werden, worauf der 2-(2-Oxo-cyclohexyl)-2-hydroxyessigsäurealkyl-ester oder substituierte 2-(2-Oxocyclohexyl)-2-hydroxyessigsäurealkyl-ester gemäß Anspruch 1, a₁) oder a₂) zu 2-Coumaron oder substituiertem 2-Coumaron weiterverarbeitet wird.

## Claims

1. Process for preparing 2-coumarone or substituted 2-coumarones, **characterized in that** cyclohexanone or substituted cyclohexanones of the formula I 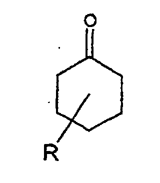 where R is H or a C₁-C₃-alkyl or alkoxy radical, are reacted with a carboxylic acylating agent of the general formula II 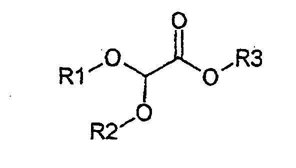 where R1 and R2 are each independently hydrogen or a C₁-C₃-alkyl radical and R3 is a C₁-C₃-alkyl radical,
a) to give alkyl 2-(2-oxocyclohexyl)-2-hydroxyacetate or substituted alkyl 2-(2-oxocyclohexyl)-2-hydroxyacetates which
a₁) are either converted directly to 2-coumarone or substituted 2-coumarones by catalytic gas phase dehydrogenation, or
a₂) dehydrated by azeotropic distillation using an acid or an acidic ion exchanger to give a mixture of alkyl 2-oxocyclohexylideneacetate and the enol lactone of 2-oxocyclohexylideneacetic acid or a mixture of substituted alkyl 2-oxocyclohexylideneacetate and the enol lactone of the substituted 2-oxocyclohexylideneacetic acid which is then in turn converted by catalytic gas phase dehydrogenation into 2-coumarone or substituted 2-coumarones, or
b) reacted directly under acidic conditions to give a mixture of alkyl 2-oxocyclohexylideneacetate and the enol lactone of 2-oxocyclohexylideneacetic acid or a mixture of substituted alkyl 2-oxocyclohexylideneacetate and the enol lactone of the substituted 2-oxocyclohexylideneacetic acid which is then in turn converted by catalytic gas phase dehydrogenation to 2-coumarone or substituted 2-coumarones.

2. Process according to Claim 1, **characterized in that** the molar ratio in step a) or b) of cyclohexanone or substituted cyclohexanones to the carboxylic acylating agent is from 100:1 to 0.5:1.

3. Process according to Claim 1, **characterized in that** the reaction temperature in step a) or b) is above 50°C.

4. Process according to Claim 1, **characterized in that** the acids used in step a₂) or b) are inorganic or organic acids or an acidic ion exchanger.

5. Process according to Claim 4, **characterized in that** the acids used are p-toluenesulfonic acid, methanesulfonic acid, hydrochloric acid, sulfuric acid or an acidic ion exchanger.

6. Process according to Claim 1, **characterized in that** aliphatic or aromatic, optionally halogenated organic solvents are used in step a₂) for azeotropic distillation and optionally in step b) for diluting the reaction mixture during the reaction.

7. Process according to Claim 1, **characterized in that** the gas phase dehydrogenation in step a₁), a₂) and b) may be carried out using either the appropriate crude intermediates or else purified intermediates.

8. Process according to Claim 1, **characterized in that** the vapour phase dehydrogenation is performed at a temperature above 140°C.

9. Process according to Claim 1, **characterized in that** the gas phase dehydrogenation is carried out using a catalyst comprising palladium or platinum which is applied to an inert, solid support having a specific surface area of more than 1 m²/g.

10. Process for preparing alkyl 2-(2-oxocyclohexyl)-2-hydroxyacetate or substituted alkyl 2-(2-oxocyclohexyl)-2-hydroxyacetates, **characterized in that** cyclohexanone or substituted cyclohexanones are reacted with the carboxylic acylating agent, whereupon the alkyl 2-(2-oxocyclohexyl)-2-hydroxyacetate or substituted methyl 2-(2-oxocyclohexyl)-2-hydroxyacetates are optionally further processed according to Claim 1, a₁) or a₂) to give 2-coumarone or substituted 2-coumarone.

## Revendications

1. Procédé de préparation de 2-coumarone ou de 2-coumarones substituées, **caractérisé en ce que** le cyclohexanone ou les cyclohexanones substituées de formule (I) 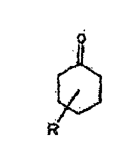 dans laquelle R est H ou un radical alcoxy ou alkyle en C₁-C₃,
avec l'agent d'acylation contenant du carboxyle de formule générale (II) 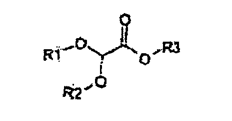 dans laquelle R1 et R2, indépendamment l'un de l'autre, peuvent être H ou un radical alkyle en C₁-C₃ et R3 représente un radical alkyle en C₁-C₃,
a) sont convertis en ester alkyle d'acide 2-(2-oxo-cyclohexyl)-2-hydroxy-acétique ou en ester alkyle d'acide 2-(2-oxo-cyclohexyl)-2-hydroxy-acétique substitué, qui
a₁) soit sont directement transformés en 2-coumarone ou 2-coumarones substituées par déshydratation en phases gazeuses catalytiques, soit
a₂) sont déshydratés par distillation azéotrope en utilisant un acide ou un échangeur d'ions acide en un mélange constitué d'ester alkyle d'acide 2-oxocyclohexylidène-acétique et de l'énol-lactone d'acide 2-oxocyclohexylidène-acétique ou un mélange constitué d'ester alkyle d'acide 2-oxocyclohexylidène-acétique substitué et de l'énol-lactone d'acide 2-oxocyclohexylidène-acétique substitué, qui est ensuite de nouveau transformé par déshydratation catalytique en phase gazeuse en 2-coumarone ou 2-coumarones substituées, ou
b) est directement converti dans des conditions acides en un mélange constitué d'ester alkyle d'acide 2-oxocyclohexylidène-acétique et de l'énol-lactone d'acide 2-oxocyclohexylidène-acétique ou en un mélange d'ester alkyle d'acide 2-oxocyclohexylidène-acétique substitué et de l'énol-lactone d'acide 2-oxocyclohexylidène-acétique substitué, qui est ensuite de nouveau transformé par déshydratation catalytique en phase gazeuse en 2-coumarone ou 2-coumarones substituées.

2. Procédé selon la revendication 1, **caractérisé en ce que**, à l'étape a) ou b), le rapport molaire entre la cyclohexanone ou la cyclohexanone substituée et l'agent d'acylation contenant du carboxyle est situé entre 100:1 et 0,5:1.

3. Procédé selon la revendication 1, **caractérisé en ce que** la température de réaction se situe au-delà de 50°C à l'étape a) ou b).

4. Procédé selon la revendication 1, **caractérisé en ce que** l'on utilise à l'étape a₂) ou b), à titre d'acides, des acides inorganiques ou organiques ou un échangeur d'ions acide.

5. Procédé selon la revendication 4, **caractérisé en ce que** l'on utilise comme acides, l'acide p-toluènesulfonique, l'acide méthanesulfonique, l'acide chlorhydrique, l'acide sulfurique ou un échangeur d'ions acide.

6. Procédé selon la revendication 1, **caractérisé en ce que** l'on utilise à l'étape a₂), pour la distillation et le cas échéant à l'étape b), pour la dilution du mélange de réaction au cours de la réaction, des solvants organiques aliphatiques ou aromatiques, le cas échéant halogénés.

7. Procédé selon la revendication 1, **caractérisé en ce que**, pour la déshydratation en phase gazeuse, on peut utiliser à l'étape a₁), a₂) et b) aussi bien les produits intermédiaires bruts correspondants que les produits intermédiaires purifiés.

8. Procédé selon la revendication 1, **caractérisé en ce que** la déshydratation en phase gazeuse est réalisée à une température supérieure à 140°C.

9. Procédé selon la revendication 1, **caractérisé en ce que**, lors de la déshydratation en phase gazeuse, on utilise un catalyseur constitué de palladium ou de platine qui est déposé sur un support inerte solide avec une surface spécifique de plus de 1 m²/g.

10. Procédé de préparation d'ester alkyle d'acide 2-(2-oxo-cyclohexyl)-2-hydroxyacétique ou d'esters alkyle d'acide 2-(2-oxo-cyclohexyl)-2-hydroxyacétique substitués, **caractérisé en ce que** la cyclohexanone ou la cyclohexanone substituée sont convertis avec l'agent d'acylation contenant du carboxyle, à la suite de quoi l'ester alkyle d'acide 2-(2-oxo-cyclohexyl)-2-hydroxyacétique ou les esters alkyle d'acide 2-(2-oxo-cyclohexyl)-2-hydroxyacétique substitués sont retransformés selon la revendication 1, a₁) ou a₂) en 2-coumarone ou 2-coumarone substituée.
